# EUROPEAN PATENT APPLICATION

(11) **EP 1 783 492 A2**
(43) Date of publication of application: **09.05.2007**
(21) Application number: 06255658.4
(22) Date of filing: 02.11.2006
(51) Int. Cl.: G01N 33/53

(54) **Tip for biomolecular reaction containing an immobilized biomolecule**

(30) Priority: 07.11.2005 JP 2005322604
(71) Applicant: Nisshinbo Industries, Inc., Chuo-ku, Tokyo 103-8650 (JP)
(72) Inventor: Kimura, Naoki, Nisshinbo Industries, Inc., Chiba-shi Chiba 267-0056 (JP)
(74) Representative: Tombling, Adrian George

(57) **Abstract**

A tip for biomolecular reaction comprises a dispensing tip and a support which is placed inside the dispensing tip and on which a biomolecule is immobilized is provided, and thereby biomolecular reaction and washing can be easily performed in a short period of time.

SEQUENCE LISTING

<110> Nisshinbo Industries, Inc.

<120> TIP FOR BIOMOLECULAR REACTION

<130>

<150> JP2005-322604
<151> 2005-11-07

<160> 12

<210> 1
<211> 10
<212> DNA
<213> Artificial Sequence

<220>
<223> Description of Artificial Sequence: synthetic oligonucleotide

<400> 1
cgcataacgc 10

<210> 2
<211> 10
<212> DNA
<213> Artificial Sequence

<220>
<223> Description of Artificial Sequence: synthetic oligonucleotide

<400> 2
cgcattacgc 10

<210> 3
<211> 10
<212> DNA
<213> Artificial Sequence

<220>
<223> Description of Artificial Sequence: synthetic oligonucleotide

<400> 3
cgcatgacgc 10

<210> 4
<211> 10
<212> DNA
<213> Artificial Sequence

<220>
<223> Description of Artificial Sequence: synthetic oligonucleotide

<400> 4
cgcatcacgc 10

<210> 5
<211> 10
<212> DNA
<213> Artificial Sequence

<220>
<223> Description of Artificial Sequence: synthetic oligonucleotide

<400> 5
gcgttatgcg 10

<210> 6
<211> 10
<212> DNA
<213> Artificial Sequence

<220>
<223> Description of Artificial Sequence: synthetic oligonucleotide

<400> 6
gcgtaatgcg 10

<210> 7

<211>  10
<212>  DNA
<213>  Artificial Sequence

<220>
<223>  Description of Artificial Sequence: synthetic oligonucleotide

<400>  7
gcgtcatgcg                                                              10

<210>  8
<211>  10
<212>  DNA
<213>  Artificial Sequence

<220>
<223>  Description of Artificial Sequence: synthetic oligonucleotide

<400>  8
gcgtgatgcg                                                              10

<210>  9
<211>  6
<212>  PRT
<213>  Artificial Sequence

<220>
<223>  Description of Artificial Sequence: synthetic peptide

<400>  9
Tyr Ile Tyr Gly Ser Phe
 1               5

<210>  10
<211>  8
<212> PRT
<213>  Artificial Sequence

<220>
<223>  Description of Artificial Sequence: synthetic peptide

<400> 10
Ala Leu Arg Arg Ala Ser Leu Gly
1               5

<210> 11
<211> 14
<212> DNA
<213> Artificial Sequence

<220>
<223> Description of Artificial Sequence: synthetic oligonucleotide

<400> 11
cccccccttt tttt                                                    14

<210> 12
<211> 15
<212> DNA
<213> Artificial Sequence

<220>
<223> Description of Artificial Sequence: synthetic oligonucleotide

<400> 12
tttttttttt ttttt                                                   15

## Description

### Technical Field

The present invention relates to a tip for biomolecular reaction.

### Background Art

Conventionally, various manual or automatic analyzers have been used in analytical tests/experiments or the like in the field of biochemistry, etc. Automatic analyzers include one that automatically takes up a liquid containing an analyte, a reagent or the like via a nozzle and treats it in accordance with a sequence of measurement procedures. An example of automatic analyzer, which is generally and widely used, includes one in which a dispensing tip is attached to one end of a nozzle, and liquid is sucked and discharged from an edge of the dispensing tip. In general, a dispensing tip is disposable and is attached to and removed from a nozzle for every use. Similarly, in the case of a manual analysis, a dispensing pipette or the like is used for sucking and discharging liquid and is disposed after each use.

Meanwhile, a microarray has been used for detection of a biological substance which can be detected by a specific reaction. A microarray is obtained by arranging a small amount of a biomolecule such as DNA on a solid phase and is used for a reaction of a sample labeled with a fluorescent dye or the like with the biomolecule on the solid phase. This enables identification and quantification of a biomolecule in a sample.
There is known a microarray equipped with an array substrate having a plurality of capillary structures in which nucleic acid probes are immobilized as a detecting part, as a method for analyzing variations in a plurality of nucleotide sequences with high accuracy and with a reduced amount of sample (JP 2004-191254 A).
Meanwhile, as a substrate for a microarray that can be applied to automatic operations for detection of a biological substance, there is known a microarray that comprises: a sample immobilizing part having a vessel-like shape comprising at least a planer bottom part on which a biological sample is immobilized at a plurality of spots and a wall part which stands from a periphery of the bottom part; and a supporting part that supports the sample immobilizing part at a predetermined height to keep the bottom part horizontal (EP1382392A).
Moreover, as a method of easily and quantitatively analyzing biomolecules by detecting two-dimensional signals, there is known a method comprising: localizing biomolecules on a solid phase; capturing a two-dimensional image on the solid phase using a scanner by scanning the solid phase two-dimensionally; and analyzing the obtained two-dimensional image data (EP1327690A).

However, in conventional techniques for detecting nucleic acids or proteins using a microarray, an operator should directly manage and perform each step of the operations, and therefore, obtained results depend on an operation ability of the operator, substantial burden and a long period of operation time are required for the operator, and there is also a risk of cross-contamination.
To solve these problems, various manufacturers sell automatic hybridization apparatuses. However, all the apparatuses require a precise water jet pump, a thermocline and the like, so it has problems such as expansion of scale of the apparatus and a high price of the apparatus itself. In addition, those apparatuses can only treat about 4 samples at one time, and simultaneous detection of many samples can only be performed by significant expansion of the scale of the apparatuses (e.g. addition of an expanded unit). There is also a problem that a step of washing a microarray requires a large amount (liter order) of washing solution for one slide.

### DISCLOSURE OF THE INVENTION

An object of the present invention is to provide means for accurately and easily performing biomolecular reaction and washing.

The inventors of the present invention have found that biomolecular reaction and washing can be easily performed in a short period of time by using, as means for solving the above-mentioned problems, a tip comprising a dispensing tip and a support that is placed inside the dispensing tip and on which a biomolecule is immobilized, thereby accomplished the present invention.
It is an object of the present invention to provide a tip for biomolecular reaction which comprises; a dispensing tip, and a support which is placed inside the dispensing tip and on which a biomolecule is immobilized.
It is a further object of the present invention to provide the above-mentioned tip for biomolecular reaction, wherein the tip is adapted to be used for an automatic dispenser.
It is a further object of the present invention to provide the above-mentioned tip for biomolecular reaction, wherein the biomolecule is selected from the group consisting of nucleic acids, proteins, enzymes, antigens, antibodies and sugars.
It is a further object of the present invention to provide the above-mentioned tip for biomolecular reaction, wherein the support is made of plastic, inorganic polymer, metal, natural polymer or ceramic.

### BRIEF DESCRIPTION OF THE DRAWINGS

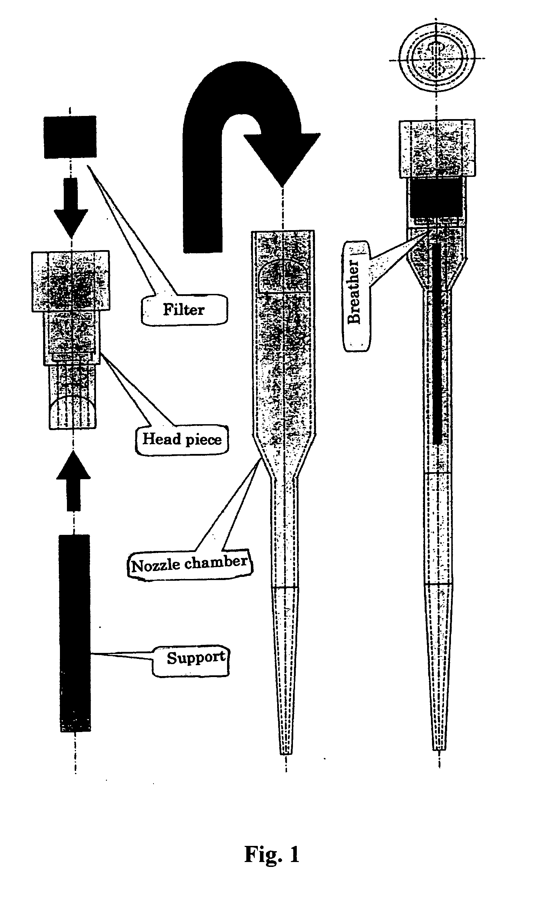

Fig. 1 is a schematic view of an example of a tip for biomolecular reaction of the present invention. The figure on the left shows a process for preparing a tip for biomolecular reaction, and the figure on the right shows a side view and a top view of the tip for biomolecular reaction.

### DESCRIPTION OF THE PREFERRED EMBODIMENTS

The present invention relates to a tip for biomolecular reaction comprising a dispensing tip and a support that is placed inside the dispensing tip and on which a biomolecule is immobilized. Hereinafter, the present invention will be described in detail.

The dispensing tip can be attached to a dispensing apparatus such as a pipette and an automatic dispensing tip, and its shape, material, size and the like are not particularly limited as long as the tip endures conditions for biomolecular reaction, general analysis of biomolecules, and the like.
In one embodiment, the dispensing tip has a tubular shape both ends of which are open, and one end of the dispensing tip is attached to a nozzle of a dispensing apparatus, while the other end is used for sucking and discharging solution such as sample solution, detection reagent solution and washing solution. In the present invention, the shape of the dispensing tip is not particularly limited. A shape of a cross-section of the center of the dispensing tip, which is perpendicular to the sucking up direction of the dispensing tip, is preferably not a circle but an elongated ellipse, an elongated rectangle or the like to reduce a volume of the inside of the dispensing tip so that volume of a reagent required for a reaction or the like is reduced and that a reagent is spread all over the support inside the dispensing tip. Further, to improve sealing performance between the dispensing tip and the nozzle of the dispensing apparatus, the dispensing tip of the present invention may have a circumferential protrusion or may be attached with a sealing member such as a head piece, a filter or an O-ring on one edge to be attached to the nozzle of the dispensing apparatus. For example, as shown in Fig. 1, a tip for biomolecular reaction of the present invention may be produced by attaching a support on which a biomolecule is immobilized to a head piece, attaching a filter to the head piece, and mounting it into a nozzle chamber of a dispensing tip.
Example of a material for a dispensing tip includes one which is insoluble in a solvent to be used for immobilization of a biomolecule and biological reaction and is solid at room temperature or at temperatures near room temperature, for example, at 0°C to 100°C. Meanwhile, a material of a dispensing tip is preferably a transparent material to detect signals easily and sensitively after a reaction on the support of the present invention and washing of the support. Furthermore, a material which can be applied to the dispensing tip of the present invention may be employed even if it is not known at present.

In the present invention, biomolecule is not particularly limited, and examples thereof include nucleic acids, proteins, enzymes, antigens, antibodies and sugars. The nucleic acids include natural or synthetic DNA (including oligodeoxynucleotides), RNA (including oligoribonucleotides) or the like, and the nucleic acids may be single-stranded or double-stranded. Meanwhile, a biomolecule may be an unknown substance to be detected or a known substance for detecting an unknown substance. Moreover, a biomolecule may be labeled with a fluorescent dye.

In the present invention, a method of immobilizing a biomolecule on the support is not particularly limited, and for example, a biomolecule may be immobilized by a chemical or physical bond or may be immobilized via a gel matrix or the like. Meanwhile, a biomolecule may be immobilized directly on the support or may be indirectly immobilized via a ligand having an ability to bind to the biomolecule (a reagent for immobilizing a biomolecule) or the like. One or more kinds of biomolecules may be immobilized on the support of the present invention. The arrangement in the case of immobilizing plural kinds of biomolecules may be appropriately selected depending on the kind of the biomolecule, detection method, application, or the like.
In addition, a biomolecule immobilized on the support or on a carrier on the support may be further linked to (elongated with) a plurality of optional biomolecules using Spot synthesis method (Heine N, Germeroth L, Schneider-Mergener J, Wenschuh H: A modular approach to the spot synthesis of 1,2,5-trisubstituted hybridizations on cellulose membranes. Tetrahedron Lett 2001, 42:227-230.), photolithography technique (Fodor SPA, Read JL, Pirrung LC, Stryer L, Lu AT, Solas D: Light-directed, spatially addressable parallel chemical synthesis. Science 1991, 251:767-773.), Fmoc method (Hasegawa K, Sha YL, Bang JK, Kawakami T, Akaji K, Aimoto S: Preparation of phosphopeptide thioesters by Fmoc- and Fmoc(2-F)-solid phase synthesis. Lett Pept Sci 2002, 8:277-284.), amidite method (JP 3129723 B), or the like.

In the present invention, a support (also referred to as a slide) is not particularly limited as long as it is capable of immobilizing a biomolecule and endures conditions for general analysis of biomolecules including hybridization. Examples thereof include one that is insoluble in a solvent to be used for immobilization, biological reactions and the like and is solid or gel at room temperature or at temperatures near room temperature, for example, at 0°C to 100°C.
Examples of the material of the support include plastics, inorganic polymers, metals, natural polymers, and ceramics.
Examples of plastics include synthetic resins such as thermoplastic resins, thermosetting resins and copolymers, and natural resins.
Specific examples of thermoplastic plastics include polycarbodiimide, ionomers such as styrene-based ionomer and olefin-based ionomer, polynorbomene, polyacetal, polyarylate, polyether ether ketone, polyethylene oxide, polyoxymethylene, polyethylene terephthalate, polycarbonate, polystyrene, polysulfone, polyparamethylstyrene, polyallylamine, polyphenylene ether, polyphenylene sulfide, polybutadiene, polybutylene terephthalate, polypropylene, polymethylpentene, polyether sulfone, polyphenylene sulfide, polyoxybenzoyl, polyoxyethylene, cellulose acetate, polydimethyl siloxane, polyisobutylene, cellulose triacetate, poly-*p*-phenylene terephthalamide, polyisoprene, polyacrylonitrile, chlorinated plastics such as polyvinyl chloride, polyethylene chloride, chlorinated polypropylene and polyvinylidene chloride, fluorinated plastics such as tetrafluoroethylene, polychlorotrifluoroethylene and polyvinylidene fluoride, nitrocellulose, polyamides such as nylon 6 and nylon 66, polyamide imide, polyimides such as thermoplastic polyimide and polyether imide, polyethylene plastics such as chlorinated polyethylene plastics, high density polyethylene plastics and low density polyethylene plastics, polyvinyl plastics such as polyvinyl chloride, polyvinyl acetate, polyparavinyl phenol, polyvinyl alcohol, polyvinyl ether, polyvinyl butyral and polyvinyl formal, liquid crystal polymers such as polyester-based liquid crystal polymer, acrylate plastics such as aminopolyacrylamide, polyacrylamide, polymethyl methacrylate, ethyl polymethacrylate and butyl polymethacrylate, and thermoplastic elastomers such as styrene-based thermoplastic elastomer, olefin-based thermoplastic elastomer, urethan-based thermoplastic elastomer, polyester-based thermoplastic elastomer, polyamide-based thermoplastic elastomer, 1,2-polybutadiene-based thermoplastic elastomer, vinyl chloride-based thermoplastic elastomer, fluorine-based thermoplastic elastomer, polyionomer-based thermoplastic elastomer, chlorinated polyethylene-based thermoplastic elastomer, and silicone-based thermoplastic elastomer.
Further, specific examples of thermosetting plastics include epoxy, polyxylene, polyguanamine, polydiallylphthalate, polyvinyl ester, polyphenol, unsaturated polyester, polyflan, polyimide, polyurethane, polymaleic acid, melamine, urea, alkyd, benzoguanamine, polycyanate and polyisocyanate.
Further, specific examples of copolymer plastics include isobutylene maleic anhydride copolymer, acrylonitrile acrylate styrene copolymer, acrylonitrile EPDM styrene copolymer, acrylonitrile styrene copolymer, acrylonitrile butadiene styrene copolymer, butadiene styrene methyl methacrylate copolymer, ethylene vinyl chloride copolymer, ethylene vinyl acetate copolymer, ethylene-ethyl acrylate copolymer, acrylonitrile-butadiene styrene copolymer, polyether ether ketone copolymer, ethylene floride polypropylene copolymer, tetrafluoroethylene perfluoroalkyl vinyl ether copolymer and tetrafluoroethylene ethylene copolymer.
Further, specific examples of natural resins include cellulose, rosin, copal, dammar, Canada balsam, elemi, sandarac, gutta percha, sumac, shellac, amber, bast fiber, leaf fiber, fruit fiber, animal hair fiber, cocoon fiber, feather fiber, chitin, chitosan, asbestos, and derivatives thereof.
Meanwhile, there may be used synthetic resins prepared by optionally adding a dye, color former, plasticizer, pigment, polymerization inhibitor, surface-modification agent, stabilizer, adhesion-imparting agent, thermosetting agent, dispersant, ultraviolet degradation inhibitor or the like to the above-mentioned synthetic resins. In addition, synthetic resins may be formed by laminating different kinds of the aforementioned synthetic resins to maintain its shape, or may be made of a single synthetic resin. Moreover, it may be a polymer alloy formed by mixing two or more kinds of the synthetic resins.
In addition, specific examples of the above-mentioned inorganic polymers include glass, crystal, carbon, silica gel and graphite.
The above-mentioned metals are not particularly limited as long as they may be used in the present invention, and preferable examples thereof include: metals selected from the elements of the I, II, III, IV, V, VI, VII, VIII groups in the second to seventh periods of the periodic system and transition elements; and alloys containing such metals. Particularly preferable examples thereof include aluminum, titanium, platinum, tungsten, molybdenum, gold, copper and nickel.
In addition, specific examples of the alloy include: a white metal composed of Cu, Ni and Zn; brass composed of Cu and Zn; bronze composed of Cu and Be; monel composed of Cu, Ni, Fe and Mn; nickel cobalt alloy composed ofNi and Co; nickel chrome alloy composed of Ni and Cr; cobalt alloy composed of Co, Ni and Cr; stainless composed of Ni, Cr and Fe; silver tungsten composed of Ag and W; b titanium composed of Ti, V and Al; ab titanium composed of Ti, V and Al; NT alloy composed of Ti and Ni; aluminium alloy composed of Al, Cu, Mg, Si, Mn and Zn; duralumin composed of Al, Cu, Si, Fe, Mn, Mg and Zn; magnesium alloy composed of Mg, Al and Zn; K24 composed of Au; K 18 composed of Au, Ag and Cu; beryllium copper composed of Cu and Be; cast iron composed of Fe, Mn, S and C; carbon steel composed of Fe, C, Si, Mn, P and S; bronze cast composed of Cu, Sn, Zn and Pb; phosphor bronze cast composed of Cu, Zn and P; brass cast composed of Cu, Zn and Pb; manganese brass composed of Cu, Zn, Mn, Fe and Al; silzin bronze cast composed of Cu, Si and Zn; aluminum bronze cast composed of Cu, Al, Fe, Ni and Mn; Elinvar composed of Ni, Cr and Mn; Elinvar Extra composed ofNi, Cr, Co and Mn; Invar composed ofNi and Fe; Super Invar composed of Fe, Ni and Co; stainless Invar composed of Fe, Co and Cr; Malottes composed of Sn, Bi and Pb; Lipowitz composed of Sn, Bi, Pb and Cd; Wood's composed of Sn, Bi, Pb and Cd; Manganin composed of Cu, Mn, Ni and Fe; Isabellin composed of Cu, Mn and Al; Constantan composed of Cu and Ni; Alcres composed of Fe, Cr and Al; Kanthal composed of Cr, Fe, Al and Co; Alumel composed ofNi and Al; magnetic material containing ferromagnetic transition elements such as Fe, Ni and Co; Permalloy composed of Fe and Ni; Alperm composed of Fe and Al; ferrite which is a complex oxide having Fe₂O₃ as a main component; Sendust composed of Fe, Si and Al; Super sendust composed of Fe, Si, Al and Ni; Alnico composed of Fe, Al, Ni and Co; hydrogen absorbing metal such as a lanthanum nickel alloy composed of La and Ni; Co-Cr-based alloy; SnO₂-based oxide; Nb-Ti alloy; damping alloy which is an alloy material which reduces or absorbs vibration, or blocks out diffusion of vibration, such as Al-Zn superplastic alloy, silent alloy and nitinol; a material for an electrode; and a material for a semiconductor such as silicon, germanium or potassium arsenide.
Meanwhile, the metals may be or may not be deposited or subjected to a plating treatment (processing) with another metal. In addition, each of the metals may be a single metal or may be laminated with a different kind of the metal to maintain its shape.
In the case of using the above-mentioned metals as a support in the present invention, the support may be made of only a metal or may be formed by laminating a metal on a nonmetallic material by means of adhesion, deposition, plating or the like.
Meanwhile, specific examples of the above-mentioned ceramics include apatite, alumina, silica, silicon carbide, silicon nitride and boron carbide.

The shape of the above-mentioned support is not particularly limited, and examples thereof include foil, plate, wafer, filter, and bead shape. The support may have a shape like a microtiter plate. In addition, an adhesive material may be applied to the back of a plate for storage of the obtained results. Meanwhile, the size of the plate is not particularly limited.

The state that a support on which a biomolecule is immobilized is placed inside a dispensing tip means that a support is present in a space inside the dispensing tip. The support may be only contained inside a dispensing tip without being immobilized as long as the support does not leave from an opening for sucking and discharging solution of the dispensing tip, or may be immobilized inside the dispensing tip. Examples of the tip of the present invention in which a support is immobilized inside a dispensing tip include one where a tip in which a support is immobilized by inserting it to a protruding part of a dispensing tip, and one where a support is immobilized by attaching it into a part such as a headpiece and mounting them into a dispensing tip as shown in figure 1. Meanwhile, number of support(s) in one dispensing tip is not particularly limited as long as a biomolecule can be analyzed and washed.

Sample solution to be sucked and discharged by the dispensing tip is not particularly limited as long as it contains a biomolecule, and the sample solution may be, for example, nucleic acid extract, protein extract, cDNA solution prepared from a biological sample, or the like. In addition, a biomolecule in sample solution may be labeled with a fluorescent dye or the like.

A method of using a tip for biomolecular reaction of the present invention is not particularly limited, and it is preferable to suck sample solution, washing solution, reaction substrate solution and the like from a nozzle so that the support is immersed into the solutions, and thereby, perform a reaction of a biomolecule immobilized on the support with a biomolecule in a sample, washing, and detection of the reaction inside the tip. For example, in the case where an automatic dispenser is used to analyze whether a biomolecule to react with a biomolecule immobilized on a support is present in a sample, the tip for biomolecular reaction of the present invention may be used according to the following method.
A biomolecule in a sample is labeled with a hapten such as biotin and digoxigenin, a fluorescent dye, or the like. A support on which a labeled biomolecule is immobilized is placed inside the dispensing tip, and the resultant tip for biomolecular reaction is set on an automatic dispenser. The automatic dispenser to be used herein is not particularly limited as long as the tip for biomolecular reaction of the present invention is applicable to the dispenser. Sample solution, washing buffer, reaction buffer and the like are dispensed in a container such as a microtiter plate. A program for the automatic dispenser is set so that the automatic dispenser sucks and discharges predetermined amounts of predetermined reagents in a predetermined order at a predetermined time, and the operations are performed to detect presence or absence of a label derived from a biomolecule in a sample that reacts with the biomolecule immobilized on the support.
As described above, it is possible to analyze whether a biomolecule that reacts with a biomolecule immobilized on a support is present in a sample. Meanwhile, detection of a label derived from a biomolecule in a sample that reacts with a biomolecule immobilized on a support enables determination of an amount of a biomolecule to react with the biomolecule immobilized on the support. Furthermore, a biomolecule to react with the biomolecule immobilized on the support can be also isolated from a sample by a similar method. Also, polymorphism of a biomolecule can be analyzed by a similar method. In addition, the above-mentioned detection can be performed according to a generally-used method depending on a kind of label. For example, fluorescence, enzyme reaction or the like may be used for the detection.
Meanwhile, a tip for biomolecular reaction of the present invention may be used in a manual dispenser by manually performing the above-mentioned processes to be automatically performed by an automatic dispenser.

As described above, in the tip for biomolecular reaction of the present invention, a reaction between biomolecules such as hybridization, washing, detection of a label and the like are performed inside a dispensing tip. Use of such a tip for biomolecular reaction reduces a burden on an operator who performs an experiment and decreases risk of cross-contamination and also enables accurate and easy reaction and washing of biomolecules without relying on an operation ability of an operator so much. In addition, use of the tip for biomolecular reaction of the present invention can minimize amounts of reagents and washing solution to be used.

The tip for biomolecular reaction of the present invention may be used in a manual dispenser but is preferably used in an automatic dispenser. Conventionally, to automatically perform a reaction such as hybridization, a large and expensive apparatus such as an automatic dispenser has been required, and in the case of simultaneously detecting many analytes, a larger apparatus has been required. In addition, large amounts of washing solution have been required for one slide. Use of the tip for biomolecular reaction of the present invention in an automatic dispenser enables accurate and easy reaction and washing of biomolecules in a shorter period of time without requiring a conventional large apparatus, and helps to reduce amounts of reagents and washing solution to be used for the reaction and washing.

Hereinafter, the present invention will be described more specifically by referring to examples. However, the present invention is not limited to the examples.

### Example 1

### Immobilization of DNA oligomer on a support, and evaluation by hybridization using a biotin-labeled DNA oligomer

Each of the oligonucleotides shown in SEQ ID NOS: 1 to 4 (10 nucleotides) was dissolved in aqueous solution of 45 mM diammonium citrate to prepare four kinds of oligonucleotide solutions (50 pmol/µl).
Each of the oligonucleotide solutions and buffer was spotted using Pixsys DNA microarray spotter (Cartesian Technologies, Inc) at three predetermined positions on a polycarbodiimide-coated slide (6.5 × 15 x 1 mm)(Nisshinbo Industries, Inc.). Each spot size was adjusted to 250 µm in diameter. Subsequently, the slide was irradiated with an ultraviolet ray of 400 mJ/cm² for 150 seconds from a 16cm-distant point using Uvstratalinker 2400 (Stratagene, central wavelength 254 nm). Thereafter, the slide was washed with shaking in water for 30 minutes and dried.
As shown in Fig. 1, four pieces of the above-mentioned slide on which the DNA oligomers were immobilized were respectively inserted to a transparent polypropylene tip (4 tips in total) (manufactured by Nakashima Works, LLC). The tips were placed at four predetermined positions in an automatic dispenser (Magtration System 12GC, Precision System Science Co., Ltd.). In addition, sterilized water (1 ml), 0.9% NaCl solution (1 ml), 1 x washing buffer (1 ml, Roche Diagnostics K.K.), AV-HRP conjugate solution (1 ml, Roche Diagnostics K.K.), and TMB Stabilized Substrate for HRP (1 ml, Promega Corporation) were separately dispensed into predetermined positions (4 wells each) of a microtiter plate (round bottom 72-well polystyrene plate, Roche Diagnostics K.K.). Further, 5'-end biotin-labeled oligonucleotides (10 nuleotides) shown in SEQ ID NOS: 5 to 8 were respectively dissolved in 80% UniHyb Hybridization Buffer (Telechem International) so as to have a final concentration of 35 ng/350 µl, and the resultant solutions were separately dispensed into predetermined positions (4 wells each) of the above-mentioned microtiter plate. The microtiter plate was placed at a predetermined position in the automatic dispenser. Subsequently, the following program for the automatic dispenser was created to perform hybridization using the above-mentioned 4 kinds of target DNAs (biotin-labeled oligonucleotides), washing, and coloring reaction by using the automatic dispenser.
(Program)
(1) Target DNA solution (300 µl) is sucked up into the tip at a flow rate of 100 µl/sec and incubated at 37°C for 30 minutes, and then, the solution is discharged from the tip at a flow rate of 100 µl/sec.
(2) 1 × washing buffer (300 µl) is sucked up into the tip at a flow rate of 100 µl/sec and incubated at room temperature for 30 seconds, and then, the solution is discharged from the tip at a flow rate of 100 µl/sec.
(3) AV-HRP conjugate solution (500 µl) is sucked up into the tip at a flow rate of 100 µl/sec and incubated at room temperature for 30 minutes, and then, the solution is discharged from the tip at a flow rate of 100 µl/sec.
(4) 1 x washing buffer (300 µl) is sucked up into the tip at a flow rate of 100 µl/sec and incubated at room temperature for 30 seconds, and then, the solution is discharged from the tip at a flow rate of 100 µl/sec.
(5) TMB Stabilized Substrate for HRP (500 µl) is sucked up into the tip at a flow rate of 100 µl/sec and incubated at room temperature for 15 minutes, and then, the solution is discharged from the tip at a flow rate of 100 µl/sec.
(6) 1 × washing buffer (300 µl) is sucked up into the tip at a flow rate of 100 µl/sec and incubated at room temperature for 30 seconds, and then, the solution is discharged from the tip at a flow rate of 100 µl/sec.
(7) 0.9% NaCl solution (500 µl) is sucked up into the tip at a flow rate of 100 µl/sec and incubated at room temperature for 30 seconds, and then, the solution is discharged from the tip at a flow rate of 100 µl/sec.
The above-mentioned program was executed using the 4 kinds of target DNAs to perform hybridization, washing, and coloring reaction. It took 1.5 hours to perform the whole procedure. The coloring reaction was performed using peroxidase-labeled streptavidin and TMB (tetramethylbenzidine).

The obtained detection results are shown in Table 1. As is evident from the results shown in Table 1, only in the case where a target DNA has a sequence completely complementary to the immobilized oligonucleotide, signal that represents specific hybridization was very clearly detected.

**Table 1**

| Target DNA | Immobilized oligonucleotides | | | |
|---|---|---|---|---|
| | SEQ ID NO: 1 | SEQ ID NO: 2 | SEQ ID NO: 3 | SEQ ID NO: 4 |
| SEQ ID NO: 5 | ⓞ | × | × | × |
| SEQ ID NO: 6 | × | ⓞ | × | × |
| SEQ ID NO: 7 | × | × | ⓞ | × |
| SEQ ID NO: 8 | × | × | × | ⓞ |

| | | | | |
|---|---|---|---|---|
| ⓞ: All signals appeared very sensitively and very clearly. ○ : All signals appeared sensitively and clearly. Δ : Some of signals appeared insensitively and unclearly. ×: All signals appeared insensitively and unclearly, or no signals appeared. | | | | |

### Example 2

### Immobilization of a peptide on a polycarbonate support, and evaluation of the peptide-immobilized support using an enzyme reaction

Peptides each having an amino acid sequence shown in SEQ ID NOS: 9 and 10 (6 and 8 residues) were synthesized using a peptide synthesizer. In accordance with a method described in JP 2004-319087 (PCT/JP2005/1882), each of the synthesized peptides and an oligonucleotide (SEQ ID NO: 11; 14 nucleotides) introduced with an amino group at its 5'-end (Glen Research Corporation) were dissolved at equal molar proportions in 0.1 M sodium hydrogen carbonate buffer (pH 8.0), and 10-fold molar excess of DS S (Disuccinimidyl suberate: Pierce Biotechnology, Inc.) dissolved in DMF (dimethylformamide) was added thereto, followed by incubation at 37°C for 2 hours, to thereby yield two kinds of solutions. Subsequently, the solutions were purified using reverse HPLC (Waters, Inc., µBondasphere, C8 300A, 3.9 x 150) and concentrated, and the concentrates were dissolved in aqueous solution of 45 mM diammonium citrate, to thereby prepare two kinds of peptide solutions (5 pmol/µl).
The peptide solutions were spotted on the surface of polycarbonate slides (Nippon Kohbunshi Co., Ltd.) using Pixsis microarray spotter (Cartesian Technologies, Inc.). Each spot size was adjusted to 0.3 mm in diameter. Subsequently, the slides were irradiated with an ultraviolet ray of 60 mJ/cm² from a 16cm-distant point for 24 seconds using Uvstratalinker 2400 (Stratagene, central wavelength 254 nm). Thereafter, the slides were washed with shaking in water for 30 minutes and dried.
As shown in Fig. 1, two pieces of the above-mentioned peptide-immobilized slide were respectively inserted into a transparent polypropylene tip (2 tips in total) (manufactured by Nakashima Works, LLC). These tips were placed at predetermined positions (2 positions each) in an automatic dispenser (Magtration System 12GC, Precision System Science Co., Ltd.). In addition, washing buffer (1 × PBS-0.2% Tween 20 solution), reaction buffer 1 containing a tyrosine kinase [2 U/50 µl p60^{c-src} kinase (Upstate), 25 mM Tris (pH 7.4), 15 mM MgCl₂, 7 mM MnCl₂, 0.5 mM EGTA, 100 µM ATP], reaction buffer 2 containing a serine kinase [serine PKA kinase (Upstate), 25 mM Tris (pH 7.4), 15 mM MgCl₂, 1 mM DTT, 2 mM EGTA, 100 µM ATP, 2 U PKA], detection buffer 1 containing an FITC-labeled anti-phosphotyrosine antibody (Sigma-Aldrich Corporation)(1 µg/100 µl antibody, 1 x PBS, 0.2% Tween 20, 1% BSA), and detection buffer 2 containing rhodamine-labeled anti-phosphotyrosine antibody (1 µg/100 µl antibody, 1 × PBS, 0.2% Tween 20, 1% BSA) were dispensed into predetermined positions (2 wells) of a microtiter plate (round bottom 72-well polystyrene plate, Roche Diagnostics K.K.). The microtiter plate was placed at a predetermined position in the automatic dispenser. Rhodamine had been conjugated to an anti-phosphorylated amino acid antibody (Cosmo Bio Co., Ltd.) in 0.1 M NaHCO₃ (pH 9.0) using rhodamine NHS (Molecular Probes). Subsequently, the following program for the automatic dispenser was created to perform phosphorylation using the kinases, washing, and detection by using the automatic dispenser.
(Program)
(1) Reaction buffer (300 µl) is sucked up in the tip at a flow rate of 100 µl/sec and incubated at 30°C for 45 minutes, and then, the solution is discharged from the tip at a flow rate of 100 µl/sec.
(2) Washing buffer (300 µl) is sucked up in the tip at a flow rate of 100 µl/sec and incubated at room temperature for 30 seconds, and then, the solution is discharged from the tip at a flow rate of 100 µl/sec.
(3) Detection buffer (500 µl) is sucked up in the tip at a flow rate of 100 µl/sec and incubated at room temperature for 45 minutes, and then, the solution is discharged from the tip at a flow rate of 100 µl/sec.
(4) Washing buffer (300 µl) is sucked up in the tip at a flow rate of 100 µl/sec and incubated at room temperature for 30 seconds, and then, the solution is discharged from the tip at a flow rate of 100 µl/sec.
The above-mentioned program was executed to perform phosphorylation, washing, and antibody reaction. It took 1.5 hours to perform the whole procedure. Subsequently, fluorescence was measured using FLA 5000 (Fuji Photo Film Co., Ltd.).

The obtained detection results are shown in Table 2. As is evident from the results shown in Table 2, only in the case of a peptide containing tyrosine, specific phosphorylation of the immobilized peptide by tyrosine kinase was very clearly detected.

**Table 2**

| Kinases | Antibodies | Immobilized peptides | |
|---|---|---|---|
| | | SEQ ID NO: 9 | SEQ ID NO: 10 |
| Tyrosine | Antiphospohtyrosine | ⓞ | × |
| (Reaction buffer 1) | (Detection buffer 1) | | |
| Serine | Antiphospohserine | × | ⓞ |
| (Reaction buffer 2) | (Detection buffer 2) | | |

| | | | |
|---|---|---|---|
| ⓞ: All signals appeared very sensitively and very clearly. ○ : All signals appeared sensitively and clearly. Δ : Some of signals appeared insensitively and unclearly. ×: All signals appeared insensitively and unclearly, or no signals appeared. | | | |

### Example 3

### Immobilization of a sugar on a support, and evaluation of sugar-immobilized support by interaction using lectin

In accordance with the method described in JP 2004-319087 (PCT/JP2005/1882), 2-aminoethyl-β-D-galactopyranoside (Mitani Sangyo Co., Ltd.), or 2-aminoethyl-β-D-glucopyranoside (Mitani Sangyo Co., Ltd.), and an oligonucleotide composed of deoxythymidylic acid and deoxycytidylic acid (SEQ ID NO: 11; 10 nucleotides) introduced with an amino group at its 5'-end (Glen Research Corporation) were dissolved in methanol:isopropyl alcohol:sterilized water:DMSO (5:5:5:1), and pH was adjusted to 8.0 by adding tributylamine solution (Wako Pure Chemical Industries, Ltd.). Subsequently, 2-fold molar excess of DSS (Pierce Biotechnology, Inc.) was added thereto, followed by incubation at 42°C for 5 hours. Subsequently, the solution was purified using reverse HPLC (Waters, Inc., µBondasphere, C8 300A, 3.9 x 150) and concentrated, and the concentrate was dissolved in aqueous solution of 45 mM diammonium citrate, to thereby prepare two kinds of sugar solutions (1 pmol/µl).
The sugar solution was spotted on a surface of a polycarbodiimide-coated slide (Nisshinbo Industries, Inc.) using Pixsis microarray spotter (Cartesian Technologies, Inc.). Each spot size was adjusted to 0.15 mm in diameter. Subsequently, the slide was irradiated with an ultraviolet ray of 120 mJ/cm² from a 16cm-distant point for 50 seconds using Uvstratalinker 2400 (Stratagene, central wavelength 254 nm). Thereafter, the slide was washed with shaking in water for 30 minutes and dried.
As shown in Fig. 1, the above-mentioned sugar-immobilized slide was inserted into a transparent polypropylene tip (1 tip) (manufactured by Nakashima Works, LLC). The tip was placed at a predetermined position (1 position) in an automatic dispenser (Magtration System 12GC, Precision System Science Co., Ltd.). In addition, reaction solution prepared by dissolving FITC-labeled lectin (derived from Sophorajaponica) in 1 × PBST (1 × PBS-0.2% Tween 20) solution containing 1% BSA at a concentration of 1 mM and washing buffer (1 × PBST solution) were dispensed at a predetermined position (1 well each) of a microtiter plate (round bottom 72-well polystyrene plate, Roche Diagnostics K.K.). FITC-labeled lectin was prepared in accordance with the method by A. McPherson et al. (McPherson, A.; Hankins, C. N.; Shannon, L. J. Biol. Chem. 1987, 262, 1791-1794). Subsequently, the following program for the automatic dispenser was created to perform interaction using the lectin, washing, and detection by using the automatic dispenser.
(Program)
(1) A lectin DNA solution (300 µl) is sucked up in the tip at a flow rate of 100 µl/sec and incubated at 30°C for 90 minutes, and then, the solution is discharged from the tip at a flow rate of 100 µl/sec.
(2) A washing buffer solution (300 µl) is sucked up in the tip at a flow rate of 100 µl/sec and incubated at room temperature for 30 seconds, and then, the solution is discharged from the tip at a flow rate of 100 µl/sec.
The above-mentioned program was executed to perform interaction with lectin, washing, and antibody reaction. It took 1.5 hours to perform the whole procedure. Subsequently, fluorescence was measured using FLA 5000 (Fuji Photo Film Co., Ltd.).

The obtained detection results are shown in Table 3. As is evident from the results shown in Table 3, only in the spots containing galactose, signal was clearly and specifically detected, which revealed that a specific interaction between lectin and sugar occurred.

**Table 3**

| Immobilized sugar | |
|---|---|
| Galactopyranoside | Glucopyranoside |
| ⓞ | × |

| | |
|---|---|
| ⓞ: All signals appeared very sensitively and very clearly. ○ : All signals appeared sensitively and clearly. Δ : Some of signals appeared insensitively and unclearly. ×: All signals appeared insensitively and unclearly, or no signals appeared. | |

### Example 4

### Immobilization of a hapten on an agarose support, and evaluation of the hapten-immobilized support by using an antibody against the hapten

Two kinds of labeled oligonucleotides which comprise biotin (compound 1) or digoxigenin (DIG: compound 2) attached via C6-alkyl spacer to 5'-end of an oligonucleotide of SEQ ID NO: 12 were synthesized by using a commercially available DNA synthesizer. Each of the labeled oligonucleotides was dissolved in aqueous solution of 45 mM diammonium citrate to prepare two kinds of hapten-labeled oligonucleotide solutions (0.5pmol/µl).
Each of the oligonucleotide solutions and buffer was spotted using Pipette Man (Gibco) at two predetermined positions on a slide (6.5 × 15 × 1 mm)(Nisshinbo Industries, Inc.) the surface of which had been coated with agarose by the method of V. Afanassiev et al. (Nucleic Acids Research, 2000, 28, e66). Each spot size was adjusted to 1mm in diameter. Subsequently, the slide was irradiated with an ultraviolet ray of 400 mJ/cm² for 150 seconds from a 16cm-distant point using Uvstratalinker 2400 (Stratagene, central wavelength 254 nm). Thereafter, the slide was washed with shaking in water for 2 minutes and dried at room temperature.
As shown in Fig. 1, one piece of the above-mentioned slide on which the hapten-labeled oligonucleotides were immobilized was inserted into a transparent polypropylene tip (manufactured by Nakashima Works, LLC). The tip was placed at a predetermined position in an automatic dispenser (Magtration System 12GC, Precision System Science Co., Ltd.). In addition, reaction solution (1 × PBST-1% BSA) containing 1mM Cy3-labeled goat anti-biotin antibody (Rockland) and washing buffer (1 × PBST solution) were separately dispensed into a predetermined position (1 well each) of a microtiter plate (round bottom 72-well polystyrene plate, Roche Diagnostics K.K.). Cy3-labeled goat anti-biotin antibody had been prepared by using Cy3 Ab Labelling kit (GE Healthcare Bioscience). Subsequently, the following program for the automatic dispenser was created to perform antigen-antibody reaction using the above-mentioned antibody, washing, and detection by using the automatic dispenser.
(Program)
(1) Antibody solution (300 µl) is sucked up into the tip at a flow rate of 100 µl/sec and incubated at 30°C for 5 minutes, and then, the solution is discharged from the tip at a flow rate of 100 µl/sec. These procedures are repeated twice.
(2) Washing buffer (300 µl) is sucked up into the tip at a flow rate of 100 µl/sec and incubated at room temperature for 30 seconds, and then, the solution is discharged from the tip at a flow rate of 100 µl/sec. These procedures are repeated four times.
The above-mentioned program was executed to perform antigen-antibody reaction and washing. It took 12 minutes to perform the whole procedure. Then, fluorescence was measured by FLA5000 (FUJIFILM).

The obtained detection results are shown in Table 4. As is evident from the results shown in Table 4, clear and specific signal was detected only at the spot containing biotin, which showed specific interaction between antigen and antibody.

**Table 4**

| Immobilized hapten | |
|---|---|
| Biotin (compound 1) | Digoxigenin (compound 2) |
| ⓞ | × |

| | |
|---|---|
| ⓞ: All signals appeared very sensitively and very clearly. ○ : All signals appeared sensitively and clearly. Δ : Some of signals appeared insensitively and unclearly. X : All signals appeared insensitively and unclearly, or no signals appeared. | |

### Example 5

### Immobilization of a hapten on a gold support, and evaluation of the hapten-immobilized support by using an antibody against the hapten

Two kinds of labeled oligonucleotides which comprise biotin (compound 1) or digoxigenin (DIG: compound 2) attached via C6-alkyl spacer to 5'-end of an oligonucleotide of SEQ ID NO: 12 were synthesized by using a commercially available DNA synthesizer. Each of the labeled oligonucleotides was dissolved in aqueous solution of 45 mM diammonium citrate to prepare two kinds of hapten-labeled oligonucleotide solutions (0.5pmol/µl).
Each of the oligonucleotide solutions and buffer was spotted using Pipette Man (Gibco) at two predetermined positions on a slide (6.5 × 15 x 1 mm)(Nisshinbo Industries, Inc.) which had been subjected to gold evaporation by Vacuum Evaporation System (VPC-260, ULVAC KIKO, Inc.). Each spot size was adjusted to 1mm in diameter. Subsequently, the slide was irradiated with an ultraviolet ray of 400 mJ/cm² for 150 seconds from a 16cm-distant point using Uvstratalinker 2400 (Stratagene, central wavelength 254 nm). Thereafter, the slide was washed with shaking in water for 2 minutes and dried at room temperature.
As shown in Fig. 1, one piece of the above-mentioned slide on which the hapten-labeled oligonucleotides were immobilized was inserted into a transparent polypropylene tip (manufactured by Nakashima Works, LLC). The tip was placed at a predetermined position in an automatic dispenser (Magtration System 12GC, Precision System Science Co., Ltd.). In addition, reaction solution (1 x PBST-1% BSA) containing 1mM Cy3-labeled goat anti-DIG antibody (Rockland) and washing buffer (1 x PBST solution) were separately dispensed into a predetermined position (1 well each) of a microtiter plate (round bottom 72-well polystyrene plate, Roche Diagnostics K.K.). Cy3-labeled goat anti-DIG antibody had been prepared by using Cy3 Ab Labelling kit (GE Healthcare Bioscience). Subsequently, the following program for the automatic dispenser was created to perform antigen-antibody reaction using the above-mentioned antibody, washing, and detection by using the automatic dispenser.
(Program)
(1) Antibody solution (300 µl) is sucked up into the tip at a flow rate of 100 µl/sec and incubated at 30°C for 5 minutes, and then, the solution is discharged from the tip at a flow rate of 100 µl/sec. These procedures are repeated four times.
(2) Washing buffer (300 µl) is sucked up into the tip at a flow rate of 100 µl/sec and incubated at room temperature for 30 seconds, and then, the solution is discharged from the tip at a flow rate of 100 µl/sec. These procedures are repeated four times.
The above-mentioned program was executed to perform antigen-antibody reaction and washing. It took 22 minutes to perform the whole procedure. Then, fluorescence was measured by FLA5000 (FUJIFILM).

The obtained detection results are shown in Table 5. As is evident from the results shown in Table 5, clear and specific signal was detected only at the spot containing digoxigenin, which showed specific interaction between antigen and antibody.

**Table 5**

| Immobilized hapten | |
|---|---|
| Biotin (compound 1) | Digoxigenin (compound 2) |
| × | ⓞ |

| | |
|---|---|
| ⓞ: All signals appeared very sensitively and very clearly. ○ : All signals appeared sensitively and clearly. Δ : Some of signals appeared insensitively and unclearly. ×: All signals appeared insensitively and unclearly, or no signals appeared. | |

### Industrial Applicability

According to the present invention, biomolecular reaction and washing can be easily performed in a short period of time. Moreover, biomolecular reaction and washing can be accurately performed.

## Claims

1. A tip for biomolecular reaction which comprises;
a dispensing tip, and
a support which is placed inside the dispensing tip and on which a biomolecule is immobilized.

2. The tip for biomolecular reaction according to Claim 1, wherein the tip is adapted to be used for an automatic dispenser.

3. The tip for biomolecular reaction according to Claim 1, wherein the biomolecule is selected from the group consisting of nucleic acids, proteins, enzymes, antigens, antibodies and sugars.

4. The tip for biomolecular reaction according to any one of Claims 1 to 3, wherein the support is made of plastic, inorganic polymer, metal, natural polymer or ceramic.
